# EUROPEAN PATENT APPLICATION

(11) **EP 1 300 155 A1**
(43) Date of publication of application: **09.04.2003**
(21) Application number: 01943852.2
(22) Date of filing: 28.06.2001
(51) Int. Cl.: A61K 38/24, A61K 38/06, A61K 9/36, A61K 47/38, A61P 25/00

(54) **ENTERIC PREPARATIONS CONTAINING PHYSIOLOGICALLY ACTIVE PEPTIDES**

(30) Priority: 11.07.2000 JP 2000209923
(71) Applicant: SHIONOGI & CO., LTD., Osaka-shi, Osaka 541-0045 (JP)
(72) Inventor: SUGITA, Katsuji c/o Shionogi & Co., Ltd., Osaka-shi, Osaka 553-0002 (JP); YOSHIKAWA, Takayoshi c/o Shionogi & Co., Ltd., Osaka-shi, Osaka 553-0002 (JP)
(74) Representative: Baverstock, Michael George Douglas
(86) International application number: JP0105543
(87) International publication number: WO02004016

(57) **Abstract**

The present invention provides an enteric coated preparation having excellent absorbability, comprising thyrotropin-releasing hormone (TRH) or derivatives thereof as a medicinally active ingredient.

## Description

### DETAILED DESCRIPTION OF THE INVENTION

### FIELD OF THE INVENTION

The present invention relates to an enteric coated preparation comprising a thyrotropin-releasing hormone (referred to hereinafter as TRH), a derivative or pharmaceutically acceptable salt or solvate thereof as a medicinally active ingredient.

### BACKGROUND ART

TRH is one of the neural peptides extracted from swine hypothalamus by Schally et al. in 1966, and a tripeptide consisting of three amino acids (L-pyroglutamyl-L-histidyl-L-prolineamide).

It is known that TRH develops not only thyrotropin or prolactin release-promoting action in hypophysis but also a variety of pharmacological actions by acting on the central nervous system (Medical Topics Series, Neural Peptide'91, Medical Review Co. Ltd). TRH derivatives having augmented action to the central nervous system generally have a structure constituted of three amino acids or derivatives thereof like TRH, and most of the derivatives have a molecular weight of about 370 to 430, described in the following reference: J. Med. Chem, 33, 2130-2137 (1990) and Japanese Patent Publication (Tokkaisho) No. S61-33197 (for example, taltirelin; TA-0910); Biomedical Research 14 (5) 317-328 (1993) and ZA7505956 (for example, montirelin; CG3703); Arzneim.-Forsch/Drug Res., 39, 297-303 (1989) and Japanese Patent Publication (Tokkaisho) No. S56-8354 (for example, posatirelin; RGH2202); Eur. J. Pharmacol. 276, 177-182 (1995) and Japanese Patent Publication (Tokkaihei) No. H3-236397 (for example, JTP-2942); Japanese Patent Publication (Tokkohei) No. H2-36574 (for example, 1-methyl-L-4,5-dihydroorotyl-L-histidyl-L-prolineamide); Japanese Patent Publication (Tokkaisho) No. S52-116465 (for example, 2,3,4,5-tetrahydro-2-oxo-L-5-furancarbonyl-L-histidyl-L-prolineamide); Japanese Patent Publication (Tokkohei) No. H3-236397 (for example, (1S,2R)-2-methyl-4-oxocyclopentyl-carbonyl-L-histidyl-L-prolineamide); Japanese Patent Publication (Tokkosho) No. S59-36612 (for example, orotyl-L-histidyl-L-prolineamide); Eur. J. Pharmacol., 271, 357 (1994) (for example, TRH-SR); Japanese Patent Publication (Tokkaisho) No. S52-3080; Eur. J. Pharmacol. 277, 63-69 (1995) and Japanese Patent Publication (Tokkaisho) No. S60-27982 (for example, azetirelin; YM14673); Japanese Patent Publication (Tokkaisho) No. S60-23326 (for example, DN1417); Japanese Patent Publication (Tokkaisho) No. S62-234029; Japanese Patent Publication (Tokkaisho) No. S56-8354; WO96/11209; WO98/08867, etc.

TRH is now commercially available as HIRTONIN (trade name) (US-3737549 (1972)). 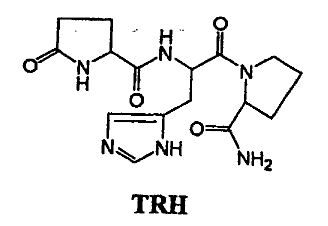 TRH or derivatives thereof generally have high stability to digestive enzymes but are highly watersoluble which results in poor absorbability from the gastrointestinal tract. Actually, most TRH derivatives have inevitably been developed or used as an injectable solution rather than an oral preparation. TRH has been approved as for example a remedy for motor ataxia by spinocerebellar degeneration, but patients with the disease must visit hospital every day to receive therapy by injection due to problems of absorbability via oral administration. However, for patients with dysbasia as predominant symptoms of spinocerebellar degeneration, to visit hospital for daily medication would cause trouble in their social life, and therefore, development of the oral preparations have been desired. In addition, TRH is of very low stability in the living body, and when used in the expectation of producing central nervous activation actions (such as antireserpine action, acetylcholine-releasing action, spontaneous motor activity-enhancing action, dopamine-enhancing action etc.), frequent administrations are necessary, which can easily produce hormonal actions (TSH (thyrotropin) hypersecretion action).

In this situation, studies have been vigorously conducted to develop TRH derivatives having even better central nervous activation actions, and the following TRH derivatives are known:

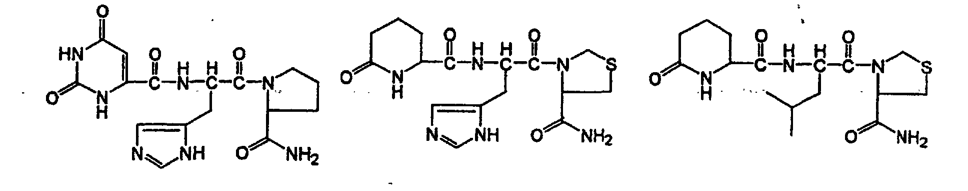

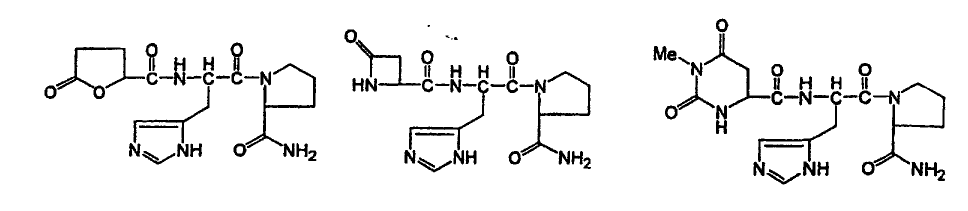

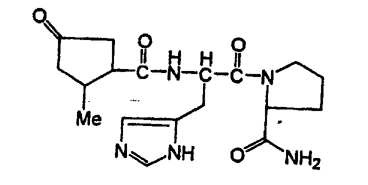

Among the above compounds, taltirelin is used to develop a remedy for spinocerebellar degeneration which is to be used as an oral preparation. However, when the preparation is orally administered to rat the bioavailability of the compound is low (3.9%) (Pharmacokinetics 12(5), 460-474 (1997)).

In addition, WO98/08867 discloses compound (1) which is a TRH derivative having superior central nervous activation actions (such as sustained acetylcholine action, antireserpine action, spontaneous motor activity-enhancing action) compared to known TRH and TRH derivatives (see the following formula). WO99/53941 also discloses an oral preparation characterized by comprising TRH derivatives, medium chain fatty acid triglycerides and if desired lecithin. This preparation enables the improved bioavailability of compound (1) in rat by improving its oral absorbability.

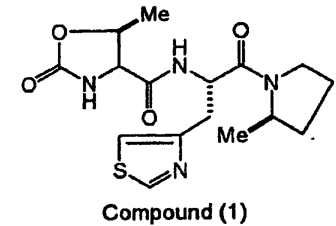

### DISCLOSURE OF THE INVENTION

The present inventors conducted the evaluation of the oral absorbability of compound (1) in a variety of animals such as rat, dog, monkey, etc., and discovered that in cynomolgus monkey decreased absorbability and markedly delayed Tmax (time to reach maximum blood concentration) was observed compared to those in rat and mouse. This reason was supposed on the basis of experiments that compound (1) was partially absorbed from the small intestine and acted at the central nerve to lead the pylorus to close, whereby excretion of compound (1) remaining in the stomach was suppressed. In clinical application in human, it is more likely that the results from cynomolgus monkey, which is a model animal more closely related to human, would be reflected. The inventors have studied hard and found that absorbability of compound (1) in cynomolgus monkey is improved by making an enteric coated preparation (tablet) of the compound, which is an accomplishment of the invention (Table 1).

Thus the present invention provides an enteric coated preparation comprising a thyrotropin-releasing hormone or derivative thereof as a medicinally active ingredient.

Specifically, the present invention provides an enteric coated preparation comprising a peptide derivative of the formula (1): 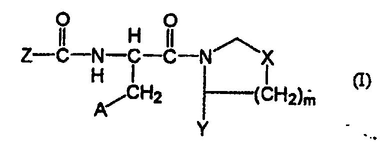
wherein A represents thiazolyl, imidazolyl, or alkyl;
X represents a single bond, oxygen atom, or sulfur atom; m is an integer of 0 to 4;
Y represents optionally substituted alkyl, optionally substituted carboxy, cyano or a group of the formula: 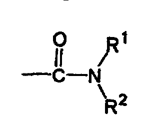 wherein R¹ and R² are identical or different and represent hydrogen atom or optionally substituted alkyl, or R¹ and R² taken together with the nitrogen atom to which they are bound form a non-aromatic heterocyclic group which may either possess oxygen atom, nitrogen atom or sulfur atom, or be optionally substituted;
Z represents a group of the formula: 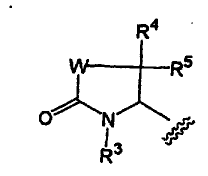 wherein R³ represents hydrogen atom, optionally substituted alkyl, optionally substituted carboxy, or optionally substituted acyl,
R⁴ and R⁵ each independently represents hydrogen atom or optionally substituted alkyl,
W represents -(CH₂)ₙ-group, wherein n is 0, 1, 2 or 3, oxygen atom, sulfur atom, or optionally substituted imino, or a group of the formula: 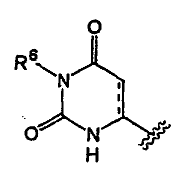 wherein R⁶ is hydrogen atom or methyl, dotted line (----) represents the presence or absence of a bond, a group of the formula:
   wherein R⁷ is hydrogen atom or methyl, or a group of the formula: 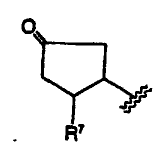 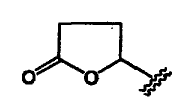 provided that the nitrogen atom on the thiazole ring of A may bind to optionally substituted alkyl or alkenyl to form a quaternary nitrogen, or a pharmaceutically acceptable salt or solvate thereof as a medicinally active ingredient.

More preferably, the present invention provides an enteric coated preparation comprising the above peptide derivative, wherein A is 4-thiazolyl or 5-thiazolyl, or a pharmaceutically acceptable salt or solvate thereof as a medicinally active ingredient.

Most preferably, the present invention provides an enteric coated preparation comprising the peptide derivative of the formula: 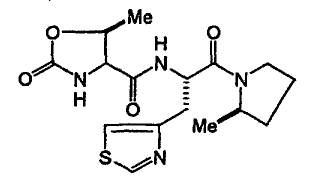 or a pharmaceutically acceptable salt or solvate thereof as a medicinally active ingredient.

More specifically, the present invention provides the above enteric coated preparation coated with an enteric base. Dosage forms of the pharmaceutical preparation include capsule, granule, or tablet, with tablet being preferred because of individual difference in, for example, the specific gravity of gastric juice or the transfer rate of the drug to the site to be absorbed.

An enteric base used in the present invention is not critical so long as the enteric base is one which does not dissolve in the stomach in order for a medicinally active component to be released in and absorbed from the intestine. The enteric base which has a dissolution pH of more than 4 and less than 7, or one which dissolved in Solution 2 of Pharmacopoeia of Japan within 60 minutes but not in Solution 1 of Pharmacopoeia of Japan, may be used. Preferably, the enteric base is a hydroxypropyl methylcellulose derivative.

The following Examples provide more detailed descriptions of the present invention for illustration purposes only, and should not be construed to limit the present invention.

### Example 1:

Absorbability of compound (1) in cynomolgus monkey by oral administration of enteric tablet comprising compound (1)

### 1) Preparation of enteric tablet

| Composition of coated tablet | |
|---|---|
| Lot No. | T8410 |
| Compound (1), bulk drug | 30.0 |
| Corn starch | 17.4 |
| HPC SL | 0.7 |
| PCS | 1.4 |
| Magnesium stearate | 0.5 |
| Total of uncoated tablet | 50.0 mg |
| HPMC2910E | 0.8 |
| HPMCAS-LF | 6.0 |
| Triethyl citrate | 0.7 |
| Talc, pulverized | 1.3 |
| Total of coating layer | 8.8 |
| Total | 58.8 mg |
| Content | 107.7-1.2% |

wherein HPC SL, PCS, HPMC2910E, and HPMCAS-LF represent hydroxypropyl cellulose SL, partially pregelatinized starch, hydroxypropylmethyl cellulose 2910E, and hydroxypropylmethyl cellulose acetate succinate-LF, respectively.

To purified water, HPMC2910E (Shin-Etsu Chemical Co., Ltd.), HPMCAS-LF (Shin-Etsu Chemical Co., Ltd.), triethyl citrate, and pulverized talc were mixed with stirring, filtered to prepare a coating solution, followed by spray coating of an uncoated tablet with compound (1) to obtain an enteric tablet.

### Aqueous coating process

Macrogol 6000 was dissolved in purified water, to which Eudragit L30D-55 (methacrylic acid copolymer) Rohm Pharma) and talc were mixed with stirring, and then filtered to prepare a coating solution. The uncoated tablet underwent spray coating to obtain an enteric film-coated tablet.

### Preparation of aqueous coating solution

CMECAQ (carboxymethylethyl cellulose) (Freund Inc.) can be used as an enteric base with dissolution pH of 5.5 in place of HPMCAS.

Glycerol caprylate was added as an elasticizer to prepare a coating solution as ethanol/water mixture.

### Organic solvent coating

CAP (cellulose acetate phthalate) (Wako Pure Chemical Industries, Ltd.) can be used as an enteric base with dissolution pH of 5.5-6.0.

CAP is not dissolved in alcohol alone and was therefore dissolved in alcohol/acetone solution to prepare a coating solution.

### Disintegration of enteric tablet

| JP13 Disintegration test | | |
|---|---|---|
| Test solution/Lot No. | | T8410 |
| Solution 1, condition after 2 hr | | Not changed |
| Mcllvaine buffer pH = | 4.0 | |
| | 4.5 | |
| | 5.0 | Not disintegrated for 70 min |
| | 5.5 | 16 min |
| | 6.0 | 8 min |

Disintegration rate of uncoated tablet: 2.0-2.3 min by water at 37°C
Solution 1: Solution 1 of Pharmacopoeia of Japan (pH 1.2)

### 2) Method of experiment

To female cynomolgus monkeys (N=5, 9-15 years old, mean body weight 3.4 ±0.4kg) which were fasted from 17.00 hours of the day before administration, uncoated tablet (Lot T8409, tablet comprising 30 mg of compound (1)) or enteric tablet (Lot T8410, dissolution pH=5.5, tablet comprising 30 mg of compound (1)) were fitted on the top of the probe for oral administration and administered at a dose of 10mg/kg of animal with 25 mL of water. Blood was periodically collected, plasma was isolated, and then plasma concentration of unchanged compound (1) was quantified with HPLC. To calculate bioavailability, the compound was separately intravenously administered at a dose of 1mg/mL/kg of animal, and calibration of the dose was performed on the basis of the obtained AUC (Area under the blood concentration-time curve) to obtain Absolute Bioavailability.

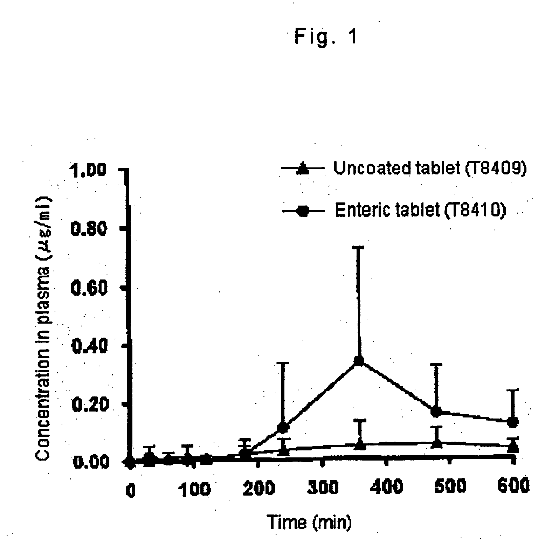

As a result, the improved oral absorbability was found in the enteric tablet comprising compound (1). Thus, in the enteric tablet comprising compound (1) coated with the enteric base having dissolution pH of 5.5, biopharmaceutical parameters such as Cmax (Maximum blood concentration), AUC, and BA (Bioavailability) were improved about three times compared with the uncoated tablet which rapidly disintegrated in water at 37°C (Table 1). Then, changes in plasma concentration for the uncoated tablet were compared with those for the enteric tablet. For the enteric tablet, the plasma concentration of compound (1) began to increase after lag time of about three hours, reached the peak in 6 hours and gradually declined while still detectable 10 hours after administration, while for the uncoated tablet the low plasma concentration was found throughout the test period (Fig. 1).

**Table 1**

| Absorbability of compound (1) in cynomolgus monkey by oral administration of enteric tablet comprising compound (1) | | | | |
|---|---|---|---|---|
| | (Cmax(µg/ml) | Tmax(min) | Auc(µg·min/ml) | BA(%) |
| Uncoated tablet (T8409) | 0.11± 0.08 | 355± 98 | 29.1± 26.3 | 3.22± 3.18 |
| Enteric tablet (T8410) | 0.35± 0.40 | 295± 186 | 100.9± 91.9 | 9.14± 10.98 |

AUC was calculate during 0-600 min
T8410: Dissolution pH = 5.5.

### BRIEF DESCRIPTION OF DRAWINGS

Fig. 1 shows absorbability of compound (1) in cynomolgus monkey by oral administration of an enteric tablet comprising compound (1).

### Example 2: Preparation of enteric tablet

Compound (1) 3g, corn starch 1.74g, and PCS (Asahi Kasei Corporation) 1.4g were mixed. The mixed powder and 1.5g of aqueous solution containing 5% HPC SL (Shin-Etsu Chemical Co., Ltd.) were kneaded together, followed by extrusion granulation by using a screen with pore size of 1 mm and then air-dried. The obtained dried granules were subjected to sieving, mixed with about 0.5 g of magnesium stearate to obtain granules, which were compression-molded to prepare uncoated tablets. To 100 g of purified water, 1.6 g of HPMC2910E (Shin-Etsu Chemical Co., Ltd.) 12 g of HPMCAS-LF (Shin-Etsu Chemical Co., Ltd.), 1.4 g of triethyl citrate, 2.6 g of pulverized talc were added and mixed to form a coating solution. An uncoated tablet was spray-coated with coating solution to prepare an enteric tablet.

## Claims

1. An enteric coated preparation comprising thyrotropin-releasing hormone or a derivative thereof as a medicinally active ingredient.

2. An enteric coated preparation comprising a peptide derivative of the formula (1): 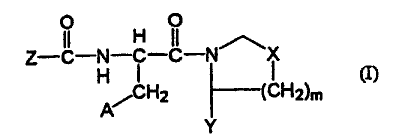
wherein A represents thiazolyl, imidazolyl, or alkyl;
X represents a single bond, oxygen atom, or sulfur atom;
m is an integer of 0 to 4;
Y represents optionally substituted alkyl, optionally substitute carboxy, cyano or a group of the formula: 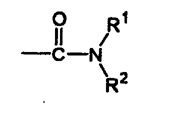
wherein R¹ and R² are identical or different and represent hydrogen atom or optionally substituted alkyl, or R¹ and R² taken together with the nitrogen atom to which they are bound form a non-aromatic heterocyclic group which may either possess oxygen atom, nitrogen atom or sulfur atom, or be optionally substituted;
Z represents a group of the formula: 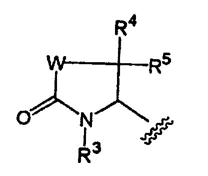 wherein R³ represents hydrogen atom, optionally substituted alkyl, optionally substituted carboxy, or optionally substituted acyl, R⁴ and R⁵ each independently represents hydrogen atom or optionally substituted alkyl, W represents
-(CH₂)ₙ-group, wherein n is 0, 1, 2 or 3, oxygen atom, sulfur atom, or optionally substituted imino, or a group of the formula: 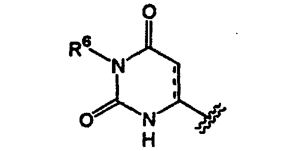
wherein R⁶ is hydrogen atom or methyl,
dotted line (----) represents the presence or absence of a bond,
a group of the formula: 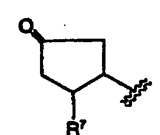 wherein R⁷ is hydrogen atom or methyl, or a group of the formula: 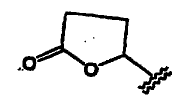 provided that the nitrogen atom on the thiazole ring of A may bind to optionally substitute alkyl or alkenyl to form a quaternary nitrogen, or a pharmaceutically acceptable salt or solvate thereof as a medicinally active ingredient.

3. An enteric coated preparation comprising a peptide derivative according to claim 2, wherein
A is 4-thiazolyl or 5-thiazolyl, or a pharmaceutically acceptable salt or solvate thereof as a medicinally active ingredient.

4. An enteric coated preparation comprising a peptide derivative of the formula: 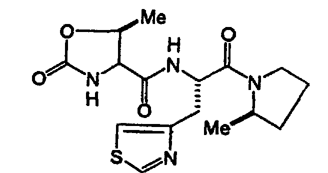 or a pharmaceutically acceptable salt or solvate thereof as a medicinally active ingredient.

5. An enteric coated preparation as claimed in any one of claims 1-4 wherein the preparation is coated with an enteric base with dissolution pH in the range of more than 4 and less than 7.

6. An enteric coated preparation as claimed in any one of claims 1-4 wherein the preparation is coated with an enteric base which is dissolved in Solution 2 of Pharmacopoeia of Japan within 60 minutes but not in Solution 1 of Pharmacopoeia of Japan.

7. An enteric coated preparation as claimed in any one of claims 1-4 wherein the preparation is coated with an enteric base which does not dissolve in the stomach.

8. An enteric coated preparation as claimed in any one of claims 5-7 wherein the enteric base comprises a hydroxypropyl methyl cellulose derivative.

9. An enteric coated preparation as claimed in any one of claims 1-8 wherein the medicinally active ingredient does not dissolve in the stomach but dissolves and is absorbed in the intestine.

10. An enteric coated preparation as claimed in any one of claims 1-9 wherein the preparation is a tablet.

11. An enteric coated preparation as claimed in any one of claims 1-10 for which absorbability is improved compared to that for a preparation which is not coated with the enteric base.
